# EUROPEAN PATENT SPECIFICATION

(11) **EP 0 797 559 B1**
(45) Date of publication and mention of the grant of the patent: **07.04.1999**
(21) Application number: 95941727.0
(22) Date of filing: 13.12.1995
(51) Int. Cl.: C07C 5/333, C07C 15/46, B01J 23/745, B01J 35/10

(54) **DEHYDROGENATION CATALYST HAVING A BIMODAL PORE SIZE DISTRIBUTION**
DEHYDRIERUNGSKATALISATOR MIT BIMODALER PORENGRÖSSENVERTEILUNG
CATALYSEUR DE DESHYDROGENATION POSSEDANT DEUX MODES DE REPARTITION DE LA GROSSEUR DES PORES

(30) Priority: 14.12.1994 US 355947
(43) Date of publication of application: 01.10.1997
(73) Proprietor: SHELL INTERNATIONALE RESEARCH MAATSCHAPPIJ B.V., 2596 HR Den Haag (NL)
(72) Inventor: HAMILTON, David, Morris, Jr., Houston, TX 77083-2925 (US)
(86) International application number: EP9505035
(87) International publication number: WO9618593

(56) References cited:
- DD-A- 245 429
- DATABASE WPI Week 8702 Derwent Publications Ltd., London, GB; AN 87-010831 & JP,A,61 268 358 (OSAKA GAS KK) , 27 November 1986

## Description

This invention relates to catalysts and processes for the dehydrogenation of hydrocarbons.

### BACKGROUND OF THE INVENTION

Iron oxide based compositions are often employed as catalysts for the dehydrogenation of hydrocarbons. Iron oxides are found in a variety of forms including the so-called red, yellow, and black forms. Yellow iron oxide is usually geothite, which is the common form of hydrated iron oxide, FeOOH. Black iron oxide is magnetite, Fe₃O₄. The red form is the anhydrous form of iron oxide known as hematite, Fe₂O₃. The red form is typically prepared by calcining the yellow form to drive off water. When the red form is prepared in this manner, the resulting particles are typically acicular or needle shape. Acicular hydrated iron oxide can also be produced by direct precipitation. The conversion of black to red iron oxides is also well known in the art. U.S. Patent No. 3,364,277 and 3,703,593 teach the use of iron oxide to prepare dehydrogenation catalysts and are incorporated herein by reference.

The form, particulate structure, and other physical characteristics assumed by an iron oxide composition can affect the physical or chemical properties of the resulting catalyst. These properties are often manifested in catalyst performance as selectivity and activity enhancements or inhibitions. Broadly speaking, the selectivity of a catalyst is its ability to produce a particular product from among two or more possible reaction products. The activity of a catalyst is its overall ability to convert reactants to products. Ordinarily, an improvement in catalyst selectivity corresponds rather directly with a worsening of catalyst activity and vice versa.

This relationship between selectivity and activity presents difficulties because one seeking higher catalyst activity must generally plan on conducting more numerous or more involved separations since an increased number of chemical species is likely. On the other hand, one seeking improved selectivity must often accept smaller yields or more numerous recycle streams. Accordingly, the art could greatly benefit from a catalyst which did not exhibit this trade off between activity and selectivity when changes in one or the other parameter are sought.

DD-A-245 429 discloses a process for the catalytic dehydrogenation of styrene, characterised in that it is performed in two subsequent reaction zones each of which contains two iron oxide based catalyst layers, one of the layers in each reaction zone having a bimodal pore size distribution displaying its maxima in the ranges of 10-70 mm and 150-250 nm and the other layer in each reaction zone having a monomodal pore size distribution and displaying its maximum in the range of 10-80 nm. The layer having the bimodal pore size distribution is stated to contribute to the selectivity of the process, and the other layer is stated to contribute to the activity.

### SUMMARY OF THE INVENTION

The instant invention presents a method for improving the selectivity of iron oxide based dehydrogenation catalysts.

In one aspect of this invention the catalyst is made from an iron-containing compound having iron oxide particles which display a bimodal pore-size distribution the maxima of which are within the ranges of 20-100 and 700-3000 nanometer respectively.

In another aspect of this invention a process for preparing iron oxide dehydrogentation catalyst is presented in which an iron-containing compound having iron oxide particles which display a bimodal pore-size distribution the maxima of which are within the ranges of 20-100 and 700-3000 nanometer respectively is combined with a potassium containing compound and water, this mixture is then formed into a pellet, and the pellet is calcined.

In yet another aspect of this invention, a process for the preparation of styrene from ethylbenzene is presented. In this process, ethylbenzene is contacted with steam and a dehydrogenation catalyst comprising iron oxide which displays a bimodal pore-size distribution the maxima of which are within the ranges of 20-100 and 700-3000 nanometer respectively.

In yet another aspect of this invention iron oxide dehydrogenation catalyst activity is improved through the use of a magnetic iron oxide therein.

### BRIEF DESCRIPTION OF THE DRAWINGS

Fig. 1 is a graphic representation of the bimodal pore size distribution of a catalyst according to this invention.

Fig. 2 is a graphic representation of the pore size distribution of a catalyst according to the prior art.

### DETAILED DESCRIPTION

This invention relates to a process for the preparation of a compound having the general formula: 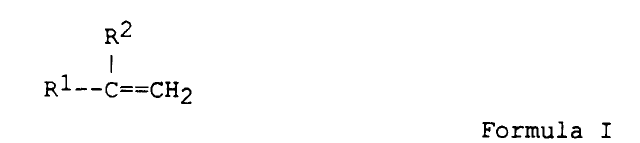 wherein R₁ and R₂ each represent an alkyl, an alkenyl or a phenyl group or a hydrogen atom, by non-oxidative dehydrogenation of a compound having the general formula: 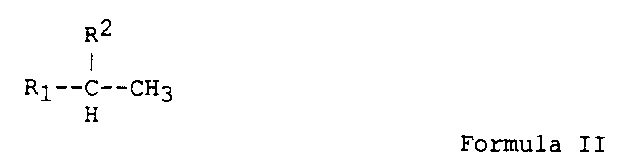 wherein R₁ and R₂ have the same meaning as in formula I, in which process a mixture comprising a compound of formula II and super-heated steam is contacted at elevated temperature with a catalyst comprising iron oxide and potassium oxide which has been prepared by combining an iron-containing compound and a potassium-containing compound to form a pellet, followed by calcination, wherein in preparing the catalyst there was used as at least part of the iron-containing compound from 10 percent to 100 percent by weight, basis of total iron-containing compounds, of iron oxide particles displaying a bimodal pore space distribution. In a preferred embodiment of this invention, the iron-containing compound comprises a magnetic iron oxide. In a most preferred embodiment of this invention the iron-containing compound comprises magnetite. As used throughout this specification, pore space distribution means the distribution of pore sizes between the maximum and the minimum as determined by the use of a Micrometrics Autopore 9220 mercury intrusion porosimeter. Furthermore, as used throughout this specification, all reference to pore size distribution, unless noted otherwise, is with respect to the pore size distribution of the finished catalyst.

R₁ in formula II may reperesent a phenyl group carrying one or more methyl groups as substituents. Preferably, R₁ is an unsubstituted phenyl group and R₂ is a hydrogen or a methyl group. Very good results have been obtained with ethylbenzene as the starting compound. The alkanes of formula II preferably have between 2 to 20 carbon atoms per molecule. It is even more preferred that they have between 3 to 8 carbon atoms such as in the case of n-butane and 2-methylbutane. The alkenes of formula II preferably have in the range of from about 4 to about 20 and particularly 4 to 8 carbon atoms per molecule. Examples include 1-butene (which can form 1,3-butadiene) and 2-methylbutane and 3-methyl-1-butene which both form isoprene. It is possible to convert n-butane with the present process via 1-butene into 1,3-butadiene and 2-methylbutane via ter.-amylenes into isoprenes.

Preferred compounds which can be produced according to this process are butadiene, alpha methyl styrene, and styrene. The use of the instant catalyst to convert ethylbenzene to styrene is particularly advantageous in that it is highly active and displays good selectivity.

A non-oxidative dehydrogenation is a dehydrogenation whereby no molecular oxygen is added. This is the type of dehydrogenation which occurs according to the process of this invention.

The term "selectivity" as used herein is the amount of compound of formula II that has been converted into compound of formula I divided by the total amount of compound of formula II that has been converted times 100. In the instant specification selectivities are typically measured at a standard rate of conversion of compound of formula II. For example, as used herein S₇₀ refers to the molar selectivity of ethylbenzene to styrene at a 70% molar conversion of ethylbenzene. The activity of a catalyst is inversely related to temperature. The more active the catalyst, the lower is the temperature that will be needed to obtain the same rate of conversion. Activities utilized in the instant specification are typically related to a given rate of conversion. For example, T₇₀ refers to the temperature at which a 70% molar conversion of ethylbenzene occurs.

The dehydrogenation process is suitably carried out using a molar ratio of steam to compound of formula II in the range of from 2 to 20 and preferably of from 5 to 13. The process is best carried out at a temperature in the range of from 400 °C to 750 °C. Even more preferred is the temperature range from 550 °C to 650 °C. The process may be carried out at atmospheric, super-atmospheric, or sub-atmospheric pressures. Atmospheric or near atmospheric pressures are preferred. It is also preferred that the process be carried out using a liquid hourly space velocity (LHSV) in the range of from 0.1 to 5.0 l/l/hr, using, for example, a tubular or radial flow reactor.

The catalyst may be used in the form of, for example, pellets, tablets, spheres, pills, saddles, trilobes, tetralobes and the like. The catalyst generally comprises 5 to 20 percent by weight of potassium oxide, from zero to 10 percent by weight of oxides of one or more promoter metals selected from the group consisting of Sc, Y, La, rare earth, Mo, W, Ca, Mg, V, Cr, Co, Ni, Mn, Cu, Zn, Cd, Al, Sn, Bi, and mixtures thereof and the balance of Fe₃O₄. Preferred promotor metals are selected from the group consisting of Ca, Mg, Mo, W, Ce, and mixtures thereof. The term "oxide" as used herein encompasses not only the single oxides, such as ferric oxide, but also mixtures of oxides such as spinels and ferrites as well as binary and other oxide mixtures. Under reaction conditions these oxides may be present in part in the form of oxidic compounds such as carbonates and bicarbonates.

The catalysts of this invention are compounded in a variety of ways. Primarily, however, they are prepared by admixing iron-containing and potassium-containing compounds which are oxides or which convert to oxides upon calcining, forming this mixture into catalyst-sized particles and calcining at elevated temperature to form a durable particle. Promotor metal-containing compounds which are oxides or which also decompose to oxides upon calcination may be admixed with the iron-containing and potassium-containing compounds. The iron-containing, potassium-containing and promoter metal containing compounds can also be denoted as oxide-providing compounds and may comprise, for example, oxides, carbonates, bicarbonates, nitrates and the like.

The catalysts are prepared with procedures which are known in the art. One method is to mix together in a muller-mixer, for example, a mixture of oxides/hydroxides/carbonates, etc., of iron, potassium and one or more optional promotor metals, adding a small amount of water, and extruding the paste to form small pellets, which are then dried at 100 °C to 300 °C and calcined at temperatures above 500 °C, preferably between 700 °C and 1000 °C. Another method is to dissolve the components together, or to make a slurry and spray dry the resultant material to form a powder, calcine the powder into oxides, and then add sufficient water to form a paste to be subsequently extruded into pellets. The pellets can then be dried and calcined. Another method involves precipitation of those materials which are precipitatable such as iron, as the resultant hydroxides, partially dewatering the resultant precipitate, adding soluble salts, for example, of potassium and promotor metals like calcium and magnesium, and then subsequently extruding, drying and calcining the extrudate. A pellet mill or pellet press can be used to form pellets. A preferred method is to first dry mix the powdered components and then mix/mull these components with sufficient water to provide an extrudatable mass. After mulling, the mixture is extruded, dried and calcined.

Generally, after the components have been formed into a catalyst particle, the particle is calcined at elevated temperature to form a durable particle. The calcining temperature will be greater than 500 °C, preferably between 700 °C and 1000 °C. Calcining atmospheres will generally be neutral (e.g. nitrogen), or oxidizing, such as oxygen or preferably air.

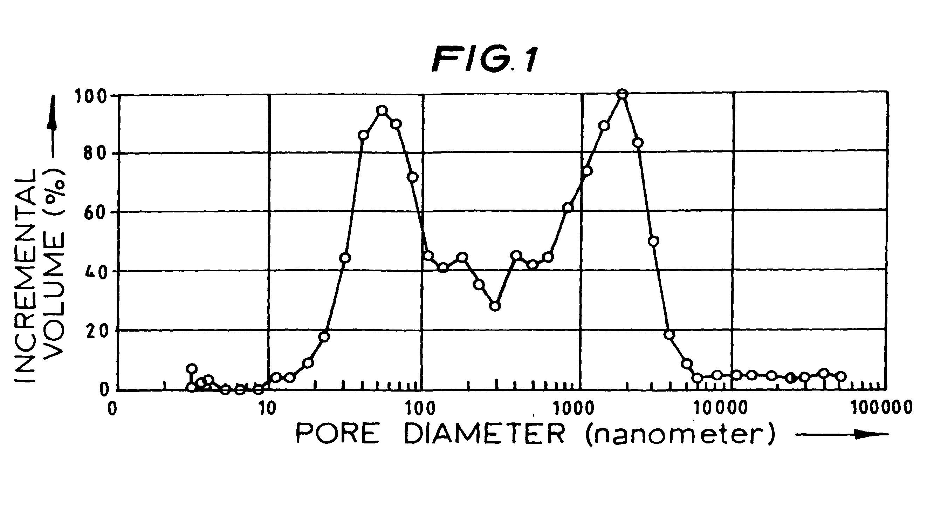

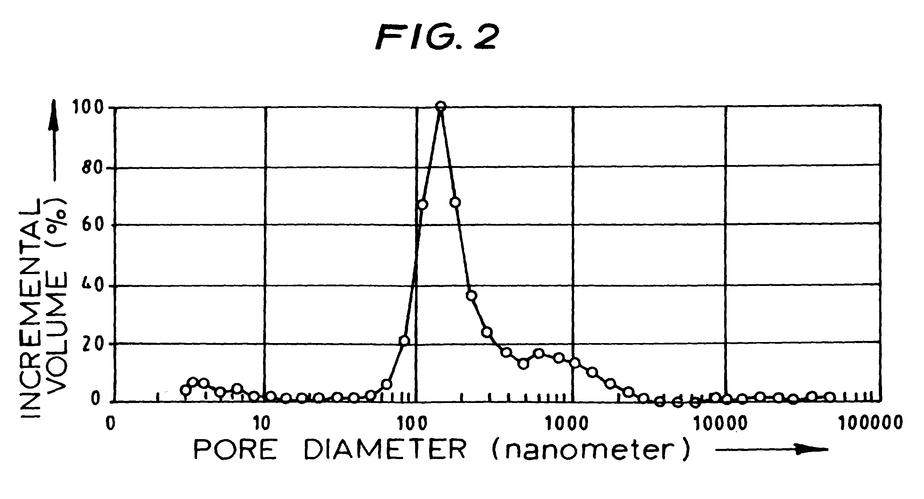

The iron oxide-providing compound that is used in the preparation of the instant catalyst is characterized by its bimodal pore size distribution. Fig. 1 is a graphical portrayal of such a bimodal distribution of a catalyst prepared according to this invention. Pore sizes ranging from less than 10 nanometer (nm) to well over 10,000 nm define the minimum and maximum values. Bimodal peaks are observed at 50 nm and 2000 nm. Fig. 2 shows a graphical representation of pore size distribution for a catalyst made from hematite derived from magnetite according to the prior art. It exhibits a single peak at a little over 100 nm. It has been found that catalyst formulated from iron oxide compounds which display a bimodal distribution also exhibit improved selectivity in the dehydrogenation of hydrocarbons. Typically, this improvement is about .8% in the catalysis of the conversion of ethylbenzene to styrene when compared to a catalyst prepared in the same manner which catalyst does not exhibit the bimodal pore size distribution.

It has been found that magnetic iron oxide compositions display the bimodal pore size distribution of the catalyst of this invention. Thus, iron oxide compositions which display noticeable magnetic properties are preferred sources of the iron oxide compositions of this invention. A most preferred source is magnetite. It is not necessary that all of the catalyst consist of magnetic iron oxide or other iron oxide displaying a bimodal pore space distribution. Noticeable selectivity improvements will be achieved if the catalyst comprise as little as 10 wt% of the iron oxide displaying bimodal pore space distribution (basis total iron oxide) with the remainder comprising a iron oxide-providing compound selected from the group consisting of goethite, hematite, moghemite, lepidocrocite, and mixtures thereof.

Without wishing to be bound to theory, it is believed that magnetic oxide particles tend to clump together in agglomerates and that mulling breaks up some, but not all, of these agglomerates into smaller sized particles. It is believed that upon calcination the smaller agglomerates will result in the formation of small catalyst pores and the unaffected agglomerates account for the larger pore sizes. Thus, the use of magnetic iron oxide based compositions can lead to the creation of catalysts having a bimodal pore size distribution.

The catalysts of this invention may also comprise any other iron compound, and can include yellow, black, and red iron oxides. Preferably, this includes an iron oxide-providing compound selected from the group consisting of geothite, hematite, maghemite, lepidocrocite and mixtures thereof. Indeed, during calcination of the catalyst most, but not all, of the Fe₃O₄ can be converted to α-Fe₂O₃ and yet the catalyst will retain its magnetic properties and display a bimodal pore size distribution. Nevertheless, it is preferred that substantially all of the iron oxide starting material be of a type that displays bimodal pore space distribution. Catalysts formulated according to this invention also generally display a higher median pore diameter and a larger pore volume than do catalysts which are similarly formulated but without iron oxide displaying a bimodal pore space distribution. Typically, median pore diameters (MPD) of the catalysts of this invention are greater than 200 nm and can even be greater than 580 nm. Pore volumes (PV) can exceed .12 cm³/g. As used throughout this specification, median pore diameter is measured by the ASTM D-4284-92, and pore volume is measured according to the ASTM D-4284-92 which is incorporated herein by reference.

The invention will be further described by the following nonlimiting examples:

### EXAMPLES

### Catalyst Preparation

Catalyst A was prepared using magnetite obtained from Harcos Pigments, Inc., (Fairview Heights, IL) as the iron oxide composition. Catalyst B was prepared from an α-Fe₂O₃ which was produced by oxidation in a rotary kiln of Fe₃O₄ obtained from Harcos Pigments, Inc..

In each case, the iron oxide was mixed with salts of promoters and water which was mixed in a muller-mixer. The resulting compositions were pelletized, dried at 170 °C for 1 hour, and calcined for 1 hour at 775 °C. 92 ml of water were used for each kg of solids in the case of Catalyst B (prior art catalyst) while 75 ml were used in the case of Catalyst A. Table 1 shows the Catalyst Formulation Data.

### Catalyst Testing

Each of the catalysts prepared as outlined above were used in the preparation of styrene from ethylbenzene under isothermal conditions in a reactor designed for continuous operation. The conditions of the catalyst test were as follows: 100 cm³ of catalyst, 600 °C reactor temperature, LHSV of 0.65 measured in liters of ethylbenzene per liter of catalyst per hour, a steam to ethylbenzene molar ratio of 10:1, and a reactor pressure of 75 kPa.

The catalyst testing results are reported in terms of T₇₀ and S₇₀ where T₇₀ is the temperature required for a given catalyst to convert 70% of the ethylbenzene feed to products and S₇₀ is the molar selectivity to product styrene. Catalyst testing results are also shown in the Table 2.

Physical properties of the respective catalysts were also measured and appear in Table 2. Pore volume, median pore diameter, and pore size distribution were measured by the methods of ASTM D-4284-92. Pore size distribution is graphically portrayed in Figs. 1 (Catalyst A) and 2 (Catalyst B).

This example illustrates the improved catalytic performance obtained by formulating iron oxide dehydrogenation catalyst such that it exhibits a bimodal pore size distribution.

**TABLE 1**

| Catalyst | Percent Loading As Oxides | | | | | mmole/mole Fe₂O₃ | | | |
|---|---|---|---|---|---|---|---|---|---|
| | Iron Oxide | K20 | CeO2 | WO3 | CaO | K | Ce | W | Ca |
| A | 79.9 | 10.7 | 5.7 | 2.6 | 1.3 | 452 | 66 | 22 | 45 |
| B | 79.9 | 10.7 | 5.7 | 2.6 | 1.3 | 452 | 66 | 22 | 45 |

**TABLE 2**

| Catalyst | T₇₀ C | S₇₀ % | Pore Vol. cm³/g | Med/Pore Diameter A | Crush lb/mm | Attr. %wt. |
|---|---|---|---|---|---|---|
| A | 598.9 | 96.1 | 0.1297 | 5817 | 6.16 | 2.59 |
| B | 594.7 | 95.3 | 0.1178 | 1808 | 5.09 | 3.20 |

## Claims

1. A dehydrogenation catalyst comprising iron oxide, which displays a bimodal pore-size distribution the maxima of which are within the ranges of 20-100 and 700-3000 nanometer respectively.

2. The catalyst of claim 1 wherein said iron-containing compound is magnetic.

3. The catalyst of claim 1 wherein said iron-containing compound comprises magnetite.

4. The catalyst of claim 1 further comprising one or more catalyst modifiers.

5. The catalyst of claim 4 wherein said modifiers are selected from the group consisting of K, Ce, Sc, Y, La, rare earth, Mo, W, Ca, Mg, V, Cr, Co, Ni, Mn, Cu, Zn, Cd, Al, Sn, Bi, and mixtures thereof.

6. A process for preparing iron oxide dehydrogenation catalyst comprising:
selecting an iron-containing compound displaying a bimodal pore-size distribution the maxima of which are within the ranges of 20-100 and 700-3000 nanometer respectively when formed into catalyst,
combining said iron-containing compound with a potassium-containing compound and water to form a mixture,
forming said mixture into a pellet, and
calcining said pellet.

7. The process of claim 6 wherein said iron-containing compound is magnetic.

8. The process of claim 7 wherein said iron-containing compound comprises magnetite.

9. The process of claim 6 further comprising the step of combining said iron-containing compound with one or more modifiers.

10. The process of claim 9 wherein said modifiers are selected from the group consisting of K, Ce, Sc, Y, La, rare earth, Mo, W, Ca, Mg, V, Cr, Co, Ni, Mn, Cu, Zn, Cd, Al, sn, Bi, and mixtures thereof.

11. A process for the preparation of styrene by the non-oxidative dehydrogenation of ethylbenzene comprising contacting ethylbenzene and steam at an elevated temperature with a dehydrogenation catalyst according to any one of claims 1-5.

## Patentansprüche

1. Dehydrierungskatalysator mit einem Gehalt an Eisenoxid, das eine bimodale Porengrößenverteilung aufweist, deren Maxima in den Bereichen 20 bis 100 beziehungsweise 700 bis 3.000 Nanometer liegen.

2. Katalysator nach Anspruch 1, worin die eisenhältige Verbindung magnetisch ist.

3. Katalysator nach Anspruch 1, worin die eisenhältige Verbindung Magnetit umfaßt.

4. Katalysator nach Anspruch 1, der weiterhin einen oder mehrere Katalysatormodifikatoren umfaßt.

5. Katalysator nach Anspruch 4, worin die Modifikatoren aus der aus K, Ce, Sc, Y, La, Seltenen Erden, Mo, W, Ca, Mg, V, Cr, Co, Ni, Mn, Cu, Zn, Cd, Al, Sn, Bi und deren Gemischen bestehenden Gruppe ausgewählt sind.

6. Verfahren zur Herstellung eines Eisenoxid-Dehydrierungskatalysators, umfassend:
Auswählen einer eisenhältigen Verbindung, die eine bimodale Porengrößenverteilung aufweist, deren Maxima in den Bereichen 20 bis 100 beziehungsweise 700 bis 3.000 Nanometer liegen, wenn sie zu einem Katalysator verformt ist,
Vereinigen dieser eisenhältigen Verbindung mit einer kaliumhältigen Verbindung und mit Wasser zur Ausbildung eines Gemisches,
Verformen dieses Gemisches zu einem Pellet und Calcinieren dieses Pellets.

7. Verfahren nach Anspruch 6, worin die eisenhältige Verbindung magnetisch ist.

8. Verfahren nach Anspruch 7, worin die eisenhältige Verbindung Magnetit umfaßt.

9. Verfahren nach Anspruch 6, das weiterhin die Stufe des Vereinigens der eisenhaltigen Verbindung mit einem oder mit mehreren Modifikatoren umfaßt.

10. Verfahren nach Anspruch 9, worin die Modifikatoren aus der aus K, Ce, Sc, Y, La, Seltenen Erden, Mo, W, Ca, Mg, V, Cr, Co, Ni, Mn, Cu, Zn, Cd, Al, Sn, Bi und deren Gemischen bestehenden Gruppe ausgewählt sind.

11. Verfahren zur Herstellung von Styrol durch nichtoxidative Dehydrierung von Ethylbenzol, umfassend das Inkontaktbringen von Ethylbenzol und Dampf bei erhöhter Temperatur mit einem Dehydrierungskatalysator nach einem der Ansprüche 1 bis 5.

## Revendications

1. Catalyseur de déshydrogénation comprenant de l'oxyde de fer, qui manifeste une distribution du calibre des pores bimodale dont les maxima varient de 20 à 100 et de 700 à 3000 nanomètres, respectivement.

2. Catalyseur suivant la revendication 1, caractérisé en ce que le composé contenant du fer précité est magnétique.

3. Catalyseur suivant la revendication 1, caractérisé en ce que le composé contenant du fer précité comprend de la magnétite.

4. Catalyseur suivant la revendication 1, caractérisé en ce qu'il comprend en outre un ou plusieurs modificateurs de catalyseur.

5. Catalyseur suivant la revendication 4, caractérisé en ce que lesdits modificateurs sont choisis dans le groupe formé par K, Ce, Sc, Y, La, des terres rares, Mo, W, Ca, Mg, V, Cr, Co, Ni, Mn, Cu, Zn, Cd, Al, Sn, Bi et leurs mélanges.

6. Procédé de préparation d'un catalyseur de déshydrogénation à base d'oxyde de fer, caractérisé en ce que :
on choisit un composé contenant du fer manifestant une distribution du calibre des pores bimodale dont les maxima se situent dans les plages de 20 à 100 et de 700 à 3000 nanomètres, respectivement, lorsqu'ils sont transformés en catalyseur,
on combine ledit composé contenant du fer à un composé contenant du potassium et de l'eau, pour former un mélange,
on transforme le mélange en un granule et
on calcine ledit granule.

7. Procédé suivant la revendication 6, caractérisé en ce que ledit composé contenant du fer est magnétique.

8. Procédé suivant la revendication 7, caractérisé en ce que ledit composé contenant du fer comprend de la magnétite.

9. Procédé suivant la revendication 6, caractérisé en ce qu'il comprend en outre l'étape de combinaison dudit composé contenant du fer et d'un ou plusieurs modificateurs.

10. Procédé suivant la revendication 9, caractérisé en ce que lesdits modificateurs sont choisis dans le groupe constitué par K, Ce, Sc, Y, La, des terres rares, Mo, W, Ca, Mg, V, Cr, Co, Ni, Mn, Cu, Zn, Cd, Al, Sn, Bi et leurs mélanges.

11. Procédé de préparation d'un styrène par la déshydrogénation non oxydante de l'éthylbenzène, caractérisé en ce que l'on met l'éthylbenzène et de la vapeur d'eau en contact à haute température avec un catalyseur de déshydrogénation suivant l'une quelconque des revendications 1 à 5.
